# EUROPÄISCHE PATENTSCHRIFT

(11) **EP 1 875 858 B1**
(45) Veröffentlichungstag und Bekanntmachung des Hinweises auf die Patenterteilung: **26.02.2014**
(21) Anmeldenummer: 07111530.7
(22) Anmeldetag: 02.07.2007
(51) Int. Cl.: A61B 5/00, A61B 5/07

(54) **Vorrichtung zur Blutungsdetektion**
Device for detecting bleeding
Dispositif destiné à la détection de saignement

(30) Priorität: 03.07.2006 DE 102006000318
(43) Veröffentlichungstag der Anmeldung: 09.01.2008
(73) Patentinhaber: NOVINEON HEALTHCARE TECHNOLOGY PARTNERS GMBH, 72074 Tübingen (DE)
(72) Erfinder: Schostek, Sebastian, 72070 Tübingen (DE); Rieber, Fabian, 70174 Stuttgart (DE); Ho, Chi-Nghia, 72074 Tübingen (DE); Schurr, Marc Oliver, Prof. Dr. med., 72074 Tübingen (DE)
(74) Vertreter: Winter, Brandl, Fürniss, Hübner, Röss, Kaiser, Polte - Partnerschaft

(56) Entgegenhaltungen:
- WO-A-2004/058041
- DE-A1- 3 743 920
- DE-A1- 10 336 734
- DE-A1-102005 032 290
- US-A- 4 181 610
- US-A- 5 131 398
- US-A- 5 833 603
- US-A1- 2002 042 562
- US-A1- 2002 111 544
- US-A1- 2003 210 390
- US-A1- 2004 176 664
- US-A1- 2004 197 771
- US-A1- 2005 154 277
- US-B1- 6 611 320

## Beschreibung

Die vorliegende Erfindung bezieht sich auf eine Vorrichtung zur Blutungsdetektion.

Blutungen im Verdauungstrakt können bei verschiedenen Gesundheitsstörungen des Verdauungstrakts auftreten, z.B. bei Speiseröhrenvarizen oder Magen- und Zwölffingerdarmgeschwüren. Selbst nach einer endoskopischen Behandlung solcher Gesundheitsstörungen treten häufig Rezidivblutungen auf, die eine schwere Komplikation darstellen. Rezidivblutungen können mehrere Stunden oder Tage nach einer Behandlung auftreten, was deren Erfassung schwierig macht. Blutungen im Verdauungstrakt können zu einer akuten Notsituation führen, da sie unbemerkt zu einem großen Blutverlust führen können. Da derzeit keine kontinuierliche Überwachungsoption zur Verfügung steht, erfolgt die Feststellung einer solchen Blutung somit oftmals zu spät.

Ein bekanntes Verfahren erfasst Blutungen durch Detektion von Farbstoffen, die vorab dem Blut zugesetzt worden sind. Allerdings funktioniert dieses Verfahren aufgrund der begrenzten Plasmahalbwertszeit des Farbstoffes nicht länger als wenige Stunden, und eignet sich daher nicht für eine postoperative Rezidivblutungsüberwachung.

Die US 5833603 offenbart eine Vorrichtung zur Erfassung der Sauerstoffsättigung zur Erfassung von Hämoglobin in Blut. Zur Erfassung wird Licht im Bereich von 660nm und Licht im Bereich von 800nm ausgesendet.

Die US 2005/0154277 A1 offenbart eine schluckbare Kapsel mit der Gastrointestinalblutungen feststellbar sind. Dabei wird Licht im Bereich von 660nm und 940nm ausgesendet.

Weitere Vorrichtungen zur Blutungsdetektion sind aus der US 2002/0111544 A1, US 2002/0042562 A1, WO 2004/058041, US 2003/0210390 A1, US 4181610 und der US 2004/0197771 A1 bekannt.

US 2004/017664 offenbart eine Vorrichtung zur Entnahme einer Blutprobe.

DE 103 36 734 A1 offenbart einen Gewebeanker für einen Endoroboter.

US 6611320 B1 offenbart ein Verfahren zur Erfassung einer Blutcharakteristik.

US 5131398 offenbart eine Vorrichtung und ein Verfahren zur Tumorerfassung.

Es ist die Aufgabe der vorliegenden Erfindung eine Vorrichtung zur Blutungsdetektion vorzusehen, die das Auftreten einer Blutung in einem Hohlorgan sicher feststellen kann.

Die Aufgabe der Erfindung wird mit einer Vorrichtung zur Blutungsdetektion gemäß Anspruch 1 gelöst.

Weitere vorteilhafte Ausgestaltungen der Erfindung sind Gegenstand der Unteransprüche.

Erfindungsgemäß wird eine Erfassungseinrichtung für ein Erfassen von Vorhandensein von Blut mittels einer Befestigungseinrichtung, vorzugsweise einem Clip, im Inneren eines Hohlorgans für eine gewisse Dauer befestigt. Dadurch dass die Erfassungseinrichtung direkt im Innenraum des Hohlorgans für eine gewisse Dauer angeordnet ist, kann eine kontinuierliche Überwachung möglicher Blutungsquellen realisiert werden.

Bevorzugt ist der Clip derart gestaltet, dass er mittels eines Endoskops in den Innenraum eines Hohlorgans transportiert werden kann, und an einer Innenwand des Hohlorgans befestigt werden kann. Ein solcher Clip ist beispielsweise aus der US 6,428,548, einer weiteren Anmeldung der Anmelderin, bekannt. Die Befestigungseinrichtung muss nicht notwendigerweise ein Clip sein, sondern kann jede beliebige Einrichtung sein, die im Inneren eines Hohlorgans befestigt bzw. verankert werden kann.

Bevorzugt ist die Befestigungseinrichtung aus einem körperverträglichen Material ausgebildet. Zusätzlich kann die Befestigungseinrichtung aus einem biologisch abbaubaren Material ausgebildet sein, das sich nach einer gewissen Zeit zersetzt. Da sich die Befestigungseinrichtung aufgrund ihrer Zersetzung nach einer gewissen Zeit von der Organinnenwand von selbst ablöst, muss die Befestigungseinrichtung nicht mittels einer Vorrichtung, wie einem Endoskop, aus dem Inneren des Hohlorgans entfernt werden, sondern wird, falls es sich bei dem Hohlorgan z.B. um den Verdauungstrakt handelt, natürlich über diesen ausgeschieden. Vorteilhafterweise ist das biologisch abbaubare Material so gewählt, dass sich die Befestigungseinrichtung aufgrund ihrer Zersetzung, unmittelbar oder kurz nach der gewünschten Beobachtungszeit von der Organinnenwand ablöst.

Die Erfassungseinrichtung misst ein Vorhandensein von Blut durch Messung von ausgesendetem Licht, das von dem Inhalt in dem Hohlorgan bzw. der Hohlorganwand absorbiert bzw. reflektiert wird. Dazu sendet die Erfassungseinrichtung Licht mit vorbestimmter Wellenlänge aus, das in dem Innenraum des Hohlorgans zumindest teilweise absorbiert oder reflektiert wird, und erfasst über einen lichtempfindlichen Sensor das reflektierte Licht. Da Blut ein charakteristisches Absorptionsspektrum aufweist, das sich vom Absorptionsspektrum des "normalen" Organinhalts unterscheidet, kann aufgrund der erfassten Reflexionen bestimmt werden, ob sich Blut im Inneren des Hohlorgans befindet oder nicht. Für die Messung des reflektierten Lichts weist die Erfassungseinrichtung mindestens einen lichtempfindlichen Sensor auf. Dieser lichtempfindliche Sensor ist vorzugsweise eine Photodiode oder ein Phototransistor. Des Weiteren weist die Erfassungsvorrichtung mindestens eine Lichtquelle auf. Bevorzugt hat die Erfassungseinrichtung aber mindestens zwei Lichtquellen, von denen mindestens eine Licht im UV-Bereich aussendet, und mindestens eine andere Licht im Rotbereich des sichtbaren Spektrums aussendet. Vorteilhafterweise ist/sind die Lichtquelle/Lichtquellen als LED(s) ausgebildet.

Bevorzugt weist die Vorrichtung zur Blutungsdetektion des weiteren eine Sendeeinheit auf, die mit der Erfassungseinrichtung verbunden ist, für ein Senden von der Erfassungseinrichtung erfasster Daten und eine Empfangseinheit für ein Empfangen der von der Sendeeinheit gesendeten Daten. Bevorzugt ist die Sendeeinheit dabei über ein datenübertragendes Kabel mit der Erfassungseinrichtung verbunden. Als Alternative kann die Erfassungseinrichtung an der Sendeinheit verankert bzw. fixiert sein oder mit dieser einstückig ausgebildet sein.

In einer vorteilhaften Ausführung ist die Erfassungseinrichtung, die Sendeeinheit und die Befestigungseinrichtung derart miteinander verbunden, dass sie eine kompakte Einheit bilden.

Die Empfangseinheit befindet sich bevorzugt außerhalb des Körpers, das heißt extrakorporal. Die Übertragung von Daten von der Sendeeinheit zu der Empfangseinheit erfolgt kabellos.

Vorteilhafterweise ist die Empfangseinheit zur Darstellung der empfangenen Signale mit einer optischen Einrichtung und/oder einer akustischen Signalisierungseinrichtung verbunden, wie ein Bildschirm oder ein Lautsprecher. Die Empfangseinheit kann auch über eine Verbindung zu einem lokalen, regionalen oder überregionalen Netzwerk verfügen, z.B. über DECT, BlueTooth, WLAN, GSM oder Satellit, um die von der Sendeeinheit empfangenen Signale zu Dritten, z.B. einem Arzt oder einer Notrufzentrale, übertragen zu können.

Bevorzugt ist die Erfassungseinrichtung und/oder die Sendeeinheit gekapselt ausgebildet. Vorzugsweise besteht diese Kapselung aus einem körperverträglichen Material.

Bevorzugt ist die Befestigungseinrichtung selbst ein Mittel zur Blutstillung einer Blutungsquelle. Dies kann beispielsweise durch Ausbilden des Befestigungseinrichtung als endoskopischer Haemostase-Clip realisiert sein.

Die Erfindung wird nachfolgend anhand eines bevorzugten Ausführungsbeispiels unter Bezugnahme auf die begleitenden Zeichnungen näher erläutert.

Fig. 1 zeigt die Vorrichtung zur Blutungsdetektion gemäß dem Ausführungsbeispiel der Erfindung in einer schematischen Ansicht.

Fig. 2 ist eine schematische Ansicht einer Erfassungseinrichtung der Vorrichtung zur Blutungsdetektion gemäß dem Ausführungsbeispiel der Erfindung.

Fig. 3 ist eine schematische Ansicht der Erfassungseinrichtung und einer Sendeeinheit der Vorrichtung zur Blutungsdetektion gemäß dem Ausführungsbeispiel der Erfindung.

Fig. 4a und 4b zeigen jeweils die Vorrichtung zur Blutungsdetektion gemäß dem Ausführungsbeispiel in Befestigungs- bzw. Erfassungsposition im Inneren eines Hohlorgans.

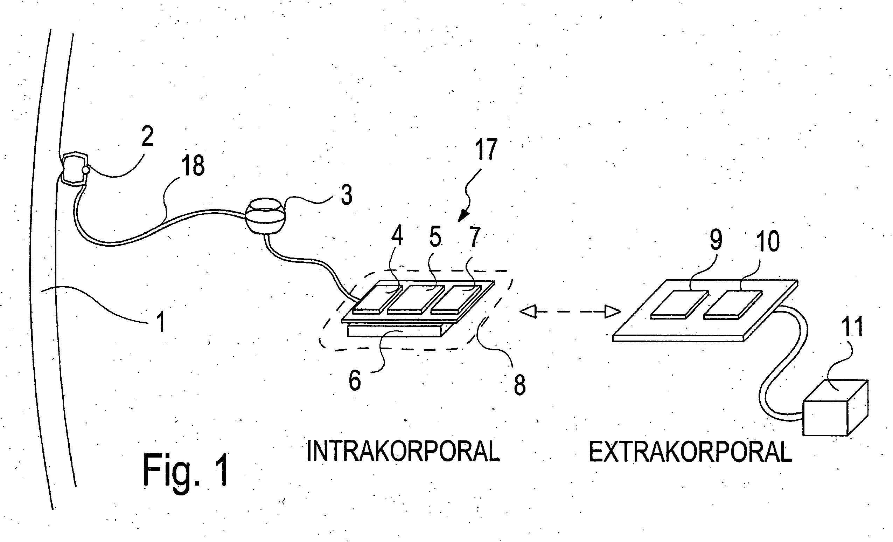

Wie aus der Fig. 1 entnommen werden kann, besteht die Vorrichtung zur Blutungsdetektion im Wesentlichen aus einem intrakorporalen Teil und einem extrakorporalen Teil. Nachfolgend wird zuerst der intrakorporale Teil beschrieben.

Der intrakorporale Teil umfasst ein Befestigungselement 2, das in dieser Ausführungsform ein Clip bzw. Anker ist, welches an einer Innenwand des Hohlorgans befestigt ist, das in diesem Ausführungsbeispiel der Verdauungstrakt ist.

Mit dem Clip 2 ist eine Erfassungseinrichtung 3 über ein Verbindungselement 18, z.B. Seil oder dergleichen, verbunden. In diesem Ausführungsbeispiel besteht die Erfassungseinrichtung 3 aus zwei Lichtquellen, von denen eine Licht im UV-Bereich und die andere Licht im Rotbereich des sichtbaren Spektrums aussendet, und einem lichtempfindlichen Sensor, wie einer Photodiode oder einem Phototransistor. Die Lichtquellen senden getaktet bzw. nacheinander Licht in den Innenraum des Hohlorgans aus, in dem das Licht absorbiert und reflektiert wird, und der lichtempfindliche Sensor erfasst das in dem Innenraum transmittierte bzw. reflektierte Licht.

In einem bevorzugten Ausführungsbeispiel ist die Befestigungseinrichtung 2 als stentförmige Struktur ausgebildet. In dieser Ausführung kann die Befestigungseinrichtung 2 die Erfassungseinrichtung 3 in einem röhrenförmigen Hohlorgan fixieren, z.B. im Duodenum zur Überwachung diffuser Blutungsquellen im Magen oder in der Speiseröhre.

Das Verbindungselement 18, das die Erfassungseinrichtung 3 mit dem Befestigungselement 2 verbindet, kann als Seil oder dergleichen ausgeführt sein. In einem Ausführungsbeispiel kann das Verbindungselement 18 aus einem sich zersetzenden Material, z.B. einem biologisch abbaubaren Material, ausgeführt sein, das sich mit der Zeit zersetzt. Die Zersetzungszeit ist je nach Organ und Anwendung so festgelegt, dass das Verbindungselement 18 erst nach Verstreichen, bevorzugt unmittelbar nach Verstreichen, einer sinnvollen Beobachtungszeit, das Befestigungselement 2 von der Erfassungseinrichtung 3 trennt. Durch diese Maßnahme kann die Erfassungseinrichtung 3 getrennt von dem Befestigungselement 2 natürlich über den Verdauungstrakt ausgeschieden werden.

Die Erfassungseinrichtung 3 ist über ein Datenübertragungskabel mit einer Sendeeinheit 17 verbunden, so dass Signale zwischen dem lichtempfindlichen Sensor und der Sendeeinheit 17 übertragen werden können. Alternativ kann die Erfassungseinrichtung auch in der Sendeeinheit 17 verankert, bzw. einstückig mit dieser ausgebildet sein.

Die Sendeeinheit 17 besteht im Wesentlichen aus einer Datenverarbeitungseinheit 4, einem Analog-/Digitalumwandler 5, einer Energiequelle 6, z.B. einer Batterie, und einem Sender 7. Die Datenverarbeitungseinheit 4 steuert die Erfassungseinrichtung 3, den A/D-Umwandler 5 und den Sender 7 bzw. wertet die von dem lichtempfindlichen Sensor erhaltenen Daten aus. Der A/D-Umwandler 5 wandelt die von der Erfassungseinrichtung 3 gesendeten, analogen Signale in digitale Signale um, und der Sender 7 übermittelt die von der Datenverarbeitungseinheit 4 ausgewerteten Daten an eine Empfangseinheit 9. Die vom Sender 7 übermittelten Daten können beispielsweise Messwerte, Statusinformationen oder Ereignis-Signale, z.B. das Ereignis des Auftretens einer Blutung, sein. In diesem Ausführungsbeispiel ist die Sendeeinheit 17 mit einer Einkapselung 8 umgeben, um die Elemente der Sendeeinheit 17 zu schützen. Vorteilhaft ist die Einkapselung aus einem körperverträglichen Material ausgebildet.

Der extrakorporale Teil besteht im Wesentlichen aus einer Empfangseinheit 9, einem Interface 10 und einer Auswerteeinheit 11. Die Empfangseinheit 9 empfängt von dem Sender 7 gesendete Daten durch eine kabellose Übertragung. Die auf diese Weise von der Empfangseinheit empfangenen Daten können mittels des Interface 10 an einer Auswerteeinheit 11 ausgewertet bzw. dargestellt werden. Die Auswerteeinheit 11 kann z.B. eine optische Einrichtung oder eine akustische Signalisierungseinrichtung sein, wie bspw. ein Display oder ein Lautsprecher. Des Weiteren können die von der Empfangseinheit empfangenen Daten mittels des Interface 10 zu Dritten, z.B. einem Arzt oder einer Notrufzentrale, übertragen werden.

Im Nachfolgenden wird nun die Funktion der Vorrichtung zur Blutungsdetektion beschrieben. Der intrakorporale Teil bestehend aus Anker 2, Erfassungseinrichtung 3 und Sendeeinheit 17 wird mittels eines Endoskops in den Verdauungstrakt eingeführt. Dort wird der Anker 2 mittels des Endoskops an einer Innenwand des Verdauungstrakts befestigt. Somit sind die Erfassungseinrichtung 3 und die Sendeeinheit 17 durch den Anker 2 im Innenraum des Verdauungstrakts befestigt. Dort erfasst die Erfassungseinrichtung 3, ob Blut in dem Verdauungstrakt vorhanden ist oder nicht. Dies funktioniert wie folgt: Die zwei Lichtquellen der Erfassungseinrichtung 3, die Licht mit vorbestimmter Wellenlänge aussenden, werden durch die Empfangseinheit 4 derart angesteuert, dass jede Lichtquelle nacheinander Licht aussendet, die eine Lichtquelle Licht im UV-Bereich, und die andere Lichtquelle Licht im Rotbereich des sichtbaren Spektrums. Das von den Lichtquellen ausgesendete Licht tritt in den Innenraum des Verdauungstrakts aus, und wird durch den Inhalt in dem Innenraum absorbiert bzw. reflektiert. Der in der Erfassungseinrichtung 3 ausgebildete lichtempfindliche Sensor in Form einer Photodiode oder Phototransistors erfasst das im Innenraum des Verdauungstrakts transmittierte bzw. reflektierte Licht, und erzeugt auf Basis des erfassten Lichts ein Sensorsignal. Es wird also für jede Lichtquelle ein Sensorsignal erzeugt. Falls sich Blut im Innenraum des Verdauungstrakts befindet, wird das von den Lichtquellen ausgesendete Licht anders absorbiert, als wenn kein Blut im Verdauungstrakt vorhanden ist, da Blut ein ganz charakteristisches Absorptionsspektrum aufweist, das sich von dem Absorptionsspektrum des "normalen" Organinhalts unterscheidet. Demzufolge unterscheidet sich das in dem Innenraum reflektierte Licht, das von dem lichtempfindlichen Sensor empfangen wird, in Abhängigkeit von Vorhandensein von Blut in dem Innenraum, und demzufolge auch die Sensorsignale, die von dem lichtempfindlichen Sensor ausgesendet werden. Somit kann aufgrund der unterschiedlichen Sensorsignale des lichtempfindlichen Sensors auf ein Vorhandensein oder nicht Vorhandensein von Blut zurückgeschlossen werden.

In einer weiteren vorteilhaften Ausführung der Erfassungseinrichtung 3 erlaubt die Ausführung der Erfassungseinrichtung 3 auch die Unterscheidung zwischen unterschiedlichen Erscheinungsformen von Blut. Unterschiedliche Erscheinungsformen von Blut sind dabei beispielsweise geronnenes Blut, venöses Blut oder arterielles Blut.

In einer weiteren vorteilhaften Ausführung hat die Erfassungseinrichtung mindestens eine Lichtquelle, die Licht im Infrarot-Bereich des Spektrums aussendet. Anhand dieser Lichtquelle kann eine Unterscheidung zwischen unterschiedlichen Erscheinungsformen von Blut ermöglicht werden.

Die von dem lichtempfindlichen Sensor ausgesendeten Sensorsignale werden dann zu der Datenverarbeitungseinheit 4 und dem AD-Umwandler 5 übertragen, wo sie ausgewertet bzw. umgewandelt werden. Anschließend sendet der Sensor 7 die ausgewerteten und umgewandelten Signale an die Empfangseinheit 9, die Bestandteil des extrakorporalen Teils ist und sich außerhalb des Körpers in Patientennähe befindet. Die von der Empfangseinheit 9 empfangenen Signale werden anschließend über eine Auswerteeinheit 11 ausgegeben, wie ein Bildschirm oder ein Lautsprecher, die über ein Interface 10 an die Empfangseinheit 9 angeschlossen werden kann. Die Auswerteeinheit 11 kann die von der Empfangseinheit 9 empfangenen Signale wahlweise auch über ein Netzwerk zu Dritten übertragen.

Auf diese Weise kann kontinuierlich überwacht werden, ob sich Blut im Inneren des Verdauungstrakts befindet. Nach Verstreichen einer sinnvollen Überwachungszeit, z.B. zwei Wochen, fällt der Anker 2 eigenständig von der Organwand ab. Der Anker kann dabei derart ausgeführt sein, dass er nach Applikation auf die Organwand nach einer sinnvollen Überwachungszeit den Halt an der Organwand verliert, z.B. durch Absterben des gegriffenen Gewebes oder einer Abstoßungsreaktion des gegriffenen Gewebes, und sich so von der Organwand ablöst.

In einer weiteren Ausführung kann der Anker 2 aus einem biologisch abbaubaren Material ausgebildet sein, das sich mit der Zeit zersetzt. Die Zersetzungszeit ist je nach Organ so festgelegt, dass der Anker 2 sich erst nach Verstreichen, bevorzugt unmittelbar nach Verstreichen, einer sinnvollen Beobachtungszeit von der Organinnenwand ablöst. Nach Ablösen des Ankers von der Organwand wird der intrakorporale Teil natürlich über den Verdauungstrakt ausgeschieden.

Alternativ kann die Vorrichtung wieder mittels eines Endoskops oder einer anderen geeigneten Vorrichtung entfernt werden.

Obwohl das Ausführungsbeispiel mit Bezug auf den Verdauungstrakt beschrieben ist, kann die vorliegende Erfindung auf ein beliebiges Hohlorgan angewendet werden. Auch kann für eine Einführung des intrakorporalen Teils und für eine Befestigung der Befestigungseinrichtung eine andere Vorrichtung als ein Endoskop verwendet werden.

Eine Vorrichtung zur Erfassung einer Blutung weist eine Erfassungseinrichtung zum Erfassen von Vorhandensein von Blut auf, die mit einer Befestigungseinrichtung verbunden ist. Die Befestigungseinrichtung wird an der Innenwand eines Hohlorgans befestigt. Die Vorrichtung hat des Weiteren eine Sendeeinheit mit der von der Erfassungseinrichtung übermittelte Daten zu einer außerhalb des Körpers angeordneten Empfangseinheit übertragen werden kann.

Die Erfassungseinrichtung sendet Licht spezifischer Wellenlängen in den Innenraum des Hohlorgans und misst das Vorhandensein von Blut vorzugsweise durch Messung der Intensität des vom Inhalt des Hohlorgans absorbierten Lichtanteils mit einem in der Erfassungseinrichtung befindlichen lichtempfindlichen Sensor. Vorzugsweise wird nacheinander Licht unterschiedlicher Wellenlängen ausgesendet und jeweils die Intensität des transmittierten Lichtanteils gemessen. Dies kann als Messung des Absorptionsspektrums bezeichnet werden. Verschiedene Arten von Organinhalt weisen unterschiedliche, spezifische Absorptionsspektren auf.

In einem bevorzugten Ausführungsbeispiel kann die Erfassungseinrichtung wahlweise Licht im Rotbereich des sichtbaren Spektrums sowie ultraviolettes Licht aussenden und mit einem lichtempfindlichen Sensor die Absorption von rotem Licht und die Absorption von ultraviolettem Licht durch den vom Licht durchdrungenen Organinhalt messen. Der lichtempfindliche Sensor kann hierfür aus einem einzelnen Photosensor, der empfindlich sowohl für rotes als auch ultraviolettes Licht ist, oder aus zwei Photosensoren bestehen, wobei der eine empfindlich für rotes Licht, der andere empfindlich für ultraviolettes Licht ist. Blut weist eine geringe Absorptionsrate für rotes Licht, aber eine hohe Absorptionsrate für ultraviolettes Licht auf. In diesem Ausführungsbeispiel führt dies dazu, dass bei Vorhandensein von Blut im vom Licht durchdrungenen Organinhalt das rote Licht relativ gering absorbiert wird, während das ultraviolette Licht relativ stark absorbiert wird. Das von der Intensität des transmittierten roten Lichts abhängige Sensorsignal ist damit gegenläufig zu dem von der Intensität des transmittierten ultravioletten Lichts abhängige Sensorsignal. Wird in der der Messung nachfolgenden Signalverarbeitung der Quotient aus dem von der Intensität des transmittierten roten Lichts abhängigen Sensorsignal und dem von der Intensität des transmittierten ultravioletten Lichts abhängigen Sensorsignal gebildet, entsteht ein einzelner Parameter, anhand dessen man auf das Vorhandensein von Blut schließen kann.

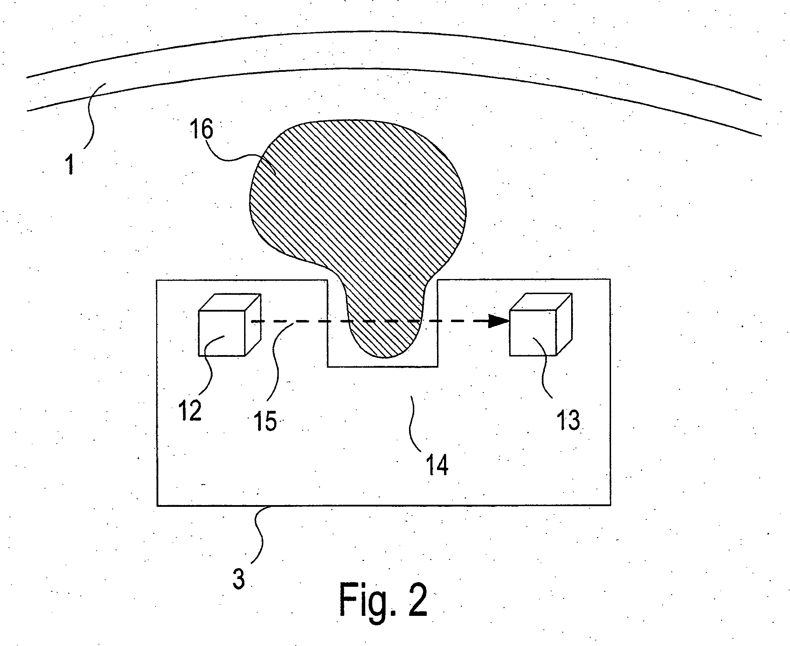

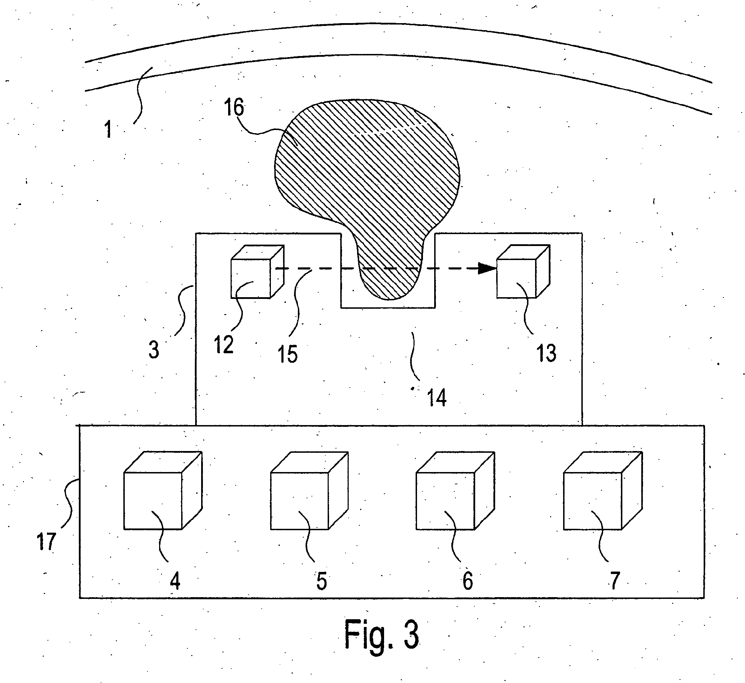

Ein bevorzugtes Ausführungsbeispiel der Erfassungseinrichtung wird nachfolgend unter Bezugnahme auf Fig. 2 und Fig. 3 näher erläutert.

Fig. 2 zeigt die Erfassungseinrichtung 3 der Vorrichtung zur Blutungsdetektion gemäß dem Ausführungsbeispiel in einer schematischen Ansicht.

Wie aus Fig. 2 entnommen werden kann, weist die Erfassungseinrichtung 3 in diesem bevorzugten Ausführungsbeispiel eine Lichtquelle 12, einen lichtempfindlichen Sensor 13 sowie einen Messspalt bzw. Aussparung 14 auf. Die Lichtquelle 12 ist in der Lage, Licht mindestens einer Wellenlänge auszusenden. Hierzu kann die Lichtquelle 12 wahlweise Licht einer oder mehrerer Wellenlängen aussenden. Vorzugsweise weist die Lichtquelle 12 hierzu eine oder mehrere LEDs auf. Die Lichtquelle 12, der lichtempfindliche Sensor 13 sowie der Messspalt 14 sind derart zueinander angeordnet, dass das von der Lichtquelle 12 ausgesendete Licht 15 aus der Erfassungseinrichtung 3 im Bereich des Messspalts 14 austritt, eine gewisse Strecke außerhalb der Erfassungseinrichtung 3 verläuft, an einer anderen Stelle des Messspalts 14 wieder in die Erfassungseinrichtung 3 eintritt und anschließend auf den lichtempfindlichen Sensor 13 fällt. Während das ausgesendete Licht 15 außerhalb der Erfassungseinrichtung 3 verläuft, kann es den Organinhalt 16 durchlaufen, der spezifische Absorptionseigenschaften aufweist. Auf diese Weise können spezifische Absorptionsspektren gemessen und Rückschlüsse auf die Art des durchleuchteten Organinhalts 16 gezogen werden.

Der Messspalt bzw. die Aussparung 14 kann hierbei verschieden ausgeführt sein, beispielsweise als Mulde, Einkerbung, Öffnung, Loch oder Spalt.

Fig. 3 zeigt die Erfassungseinrichtung 3 sowie die Sendeeinheit 17 der Vorrichtung zur Blutungsdetektion gemäß dem Ausführungsbeispiel in einer schematischen Ansicht.

Wie aus Fig. 3 entnommen werden kann, ist die Erfassungseinrichtung 3 in diesem Ausführungsbeispiel einstückig mit der Sendeeinheit 17 ausgeführt. Die Sendeeinheit 17 weist eine Datenverarbeitungseinheit 4, einen Analog-/Digitalumwandler 5, eine Energiequelle 6 sowie einen Sender 7 auf.

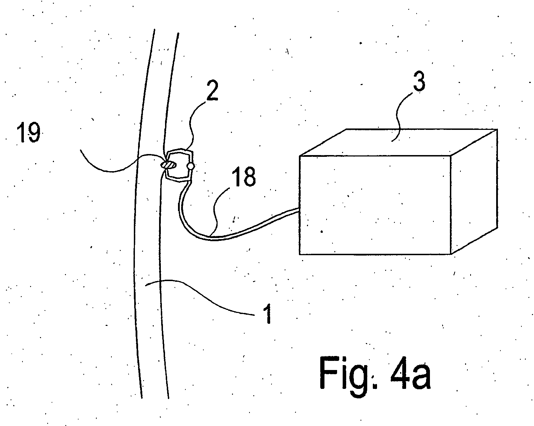

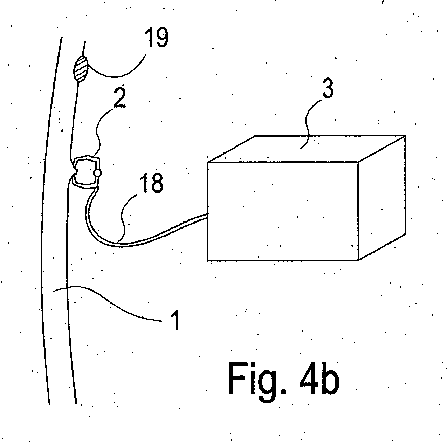

Bevorzugte Anwendungsbeispiele der Vorrichtung zur Blutungsdetektion werden nachfolgend unter Bezugnahme auf Fig. 4a und Fig. 4b näher erläutert.

Fig. 4a zeigt die Befestigungseinrichtung 2, das Verbindungselement 18, die Erfassungseinrichtung 3 sowie die Hohlorganwand 1. Die Hohlorganwand 1 weist hierbei eine potenzielle Blutungsquelle 19 auf. In diesem Anwendungsbeispiel ist die Befestigungseinrichtung 2 auf der potenziellen Blutungsquelle 19 an der Hohlorganwand 1 befestigt und dient daher sowohl zur Behandlung der potenziellen Blutungsquelle 19 als auch zur Befestigung der Erfassungseinrichtung 3. Die potenzielle Blutungsquelle 19 kann hierbei z.B. ein Magen- oder Zwölffingerdarmgeschwür sein.

Fig. 4b zeigt die Befestigungseinrichtung 2, das Verbindungselement 18, die Erfassungseinrichtung 3 sowie die Hohlorganwand 1. Die Hohlorganwand 1 weist hierbei eine potenzielle Blutungsquelle 19 auf. In diesem Anwendungsbeispiel ist die Befestigungseinrichtung 2 an einem anderen Ort als die Blutungsquelle 19 an der Hohlorganwand 1 befestigt und dient daher zur Befestigung der Erfassungseinrichtung 3 in der Nähe der potenziellen Blutungsquelle 19 und nicht zur Behandlung der potenziellen Blutungsquelle 19. Die potenzielle Blutungsquelle kann hierbei eine stark blutungsgefährdete Struktur sein, z.B. eine behandelte oder unbehandelte Varize in Speiseröhre bzw. Magen oder ein behandeltes oder unbehandeltes Magen- bzw. Zwölffingerdarmgeschwür. In diesem Ausführungsbeispiel kann die Befestigungseinrichtung 2 auch als stentförmige Struktur ausgebildet sein.

## Patentansprüche

1. Vorrichtung zur Blutungsdetektion mit
einer Befestigungseinrichtung (2), die im Inneren eines Hohlorgans befestigt werden kann,
einer mit der Befestigungseinrichtung (2) verbundenen Erfassungseinrichtung (3) für ein Erfassen von Vorhandensein von Blut,
einer Lichtquelle (12), die derart ausgebildet ist, Licht im ultravioletten Bereich und im roten Bereich des sichtbaren Spektrums auszusenden,
mindestens einem lichtempfindlichen Sensor (13), der derart ausgebildet ist, Licht in diesem ultravioletten Bereich und in diesem roten Bereich des sichtbaren Spektrums zu messen und je ein Sensorsignal in Abhängigkeit von der gemessenen Lichtintensität in den beiden Bereichen auszugeben, und
einer Signalverarbeitungseinheit, die derart ausgebildet ist, einen Quotienten aus den beiden Sensorsignalen zu berechnen, wobei der Quotient ein Parameter ist, der über das Vorhandensein von Blut entscheidet.

2. Vorrichtung zur Blutungsdetektion gemäß Anspruch 1, wobei die Lichtquelle (12) das Licht im ultravioletten Bereich und im roten Bereich des sichtbaren Lichts nacheinander aussendet.

3. Vorrichtung zur Blutungsdetektion gemäß Anspruch 1, wobei zwei lichtempfindliche Sensoren (13) vorgesehen sind, von denen einer nur Licht in dem ultravioletten Bereich und der andere nur Licht in dem roten Bereich des sichtbaren Lichts misst.

4. Vorrichtung zur Blutungsdetektion gemäß Anspruch 1, die des Weiteren eine Sendeeinheit (4, 5, 6, 7) für ein Senden von der Erfassungseinrichtung (3) erfassten Daten und eine Empfangseinheit (9) für ein Empfangen der von der Sendeeinheit (7) gesendeten Daten aufweist.

5. Vorrichtung zur Blutungsdetektion gemäß Anspruch 1 oder 4, **dadurch gekennzeichnet, dass** die Sendeinheit (4, 5, 6, 7) gekapselt ausgebildet ist.

6. Vorrichtung zur Blutungsdetektion gemäß Anspruch 4 oder 5, **dadurch gekennzeichnet, dass** die Sendeeinheit (4, 5, 6, 7) kabellos Daten zu der Empfangseinheit (9) übertragt.

7. Vorrichtung zur Blutungsdetektion gemäß einem der Ansprüche 1 bis 6, **dadurch gekennzeichnet, dass** die Befestigungseinrichtung (2) ein endoskopischer Clip oder Anker ist.

8. Vorrichtung zur Blutungsdetektion gemäß einem der Ansprüche 1 bis 6, **dadurch gekennzeichnet, dass** die Befestigungseinrichtung (2) eine stentartige Struktur ist.

9. Vorrichtung zur Blutungsdetektion gemäß einem der Ansprüche 1 bis 8, **dadurch gekennzeichnet, dass** die Befestigungseinrichtung (2) aus einem biologisch abbaubaren Material ausgebildet ist.

10. Vorrichtung zur Blutungsdetektion gemäß einem der Ansprüche 1 bis 9, **dadurch gekennzeichnet, dass** die Befestigungseinrichtung (2) über ein Verbindungselement (18) mit der Erfassungseinrichtung (3) verbunden ist.

11. Vorrichtung zur Blutungsdetektion gemäß Anspruch 10, **dadurch gekennzeichnet, dass** das Verbindungselement (18) aus einem biologisch abbaubaren Material ausgebildet ist.

12. Vorrichtung zur Blutungsdetektion gemäß Anspruch 1, **dadurch gekennzeichnet, dass** die Lichtquelle eine LED ist.

13. Vorrichtung zur Blutungsdetektion gemäß einem der Ansprüche 1 bis 12, **dadurch gekennzeichnet, dass** die Erfassungseinrichtung (3) einen Messspalt (14) aufweist.

14. Vorrichtung zur Blutungsdetektion gemäß Anspruch 13, **dadurch gekennzeichnet, dass** das von der Lichtquelle ausgesendete Licht im Bereich des Messspalts (14) den Innenraun des Hohlorgans durchlaufen kann.

15. Vorrichtung zur Blutungsdetektion gemäß einem der Ansprüche 1 bis 14, **dadurch gekennzeichnet, dass** die Befestigungseinrichtung (2) ein Mittel zur Blutstillung einer Blutungsquelle darstellt, beispielsweise ein endoskopischer Haemostase-Clip ist.

## Claims

1. A device for hemorrhage detection, comprising
a fixing means (2) mountable inside a hollow organ,
a detecting means (3) connected to the fixing means (2) for detecting a presence of blood,
a light source (12) configured to emit light in the UV range and in the red range of the visible spectrum,
at least one photosensitive sensor (13) configured to measure light in the UV range and in the red range of the visible spectrum, and to output a sensor signal each, depending on the measured intensity of the light in the two ranges, and
a signal processing unit configured to calculate a quotient from the two sensor signals, wherein the quotient is a parameter which decides on the presence of blood.

2. The device for hemorrhage detection according to claim 1, wherein the light source (12) emits light successively in the UV range and in the red range of the visible light.

3. The device for hemorrhage detection according to claim 1, wherein two photosensitive sensors (13) are provided of which one exclusively measures light in the UV range and the other one exclusively in the red range of the visible light.

4. The device for hemorrhage detection according to claim 1, further comprising a transmitting unit (4, 5, 6, 7) for sending data detected by the detecting means (3), and a receiving unit (9) for receiving the data sent by the transmitting unit (7).

5. The device for hemorrhage detection according to claim 1 or 4, **characterized in that** the transmitting unit (4, 5, 6, 7) is formed to be encapsulated.

6. The device for hemorrhage detection according to claim 4 or 5, **characterized in that** the transmitting unit (4, 5, 6, 7) transmits data to the receiving unit (9) wirelessly.

7. The device for hemorrhage detection according to any one of claims 1 to 6, **characterized in that** the fixing means (2) is an endoscopic clip or anchor.

8. The device for hemorrhage detection according to any one of claims 1 to 6, **characterized in that** the fixing means (2) is a stent-like structure.

9. The device for hemorrhage detection according to any one of claims 1 to 8, **characterized in that** the fixing means (2) is made of a biodegradable material.

10. The device for hemorrhage detection according to any one of claims 1 to 9, **characterized in that** the fixing means (2) is connected to the detecting means (3) via a connecting member (18).

11. The device for hemorrhage detection according to claim 10, **characterized in that** the connecting member is made of a biodegradable material.

12. The device for hemorrhage detection according to claim 1, **characterized in that** the light source is an LED.

13. The device for hemorrhage detection according to any one of claims 1 to 12, **characterized in that** the detecting means (3) comprises a measuring gap (14).

14. The device for hemorrhage detection according to claim 13, **characterized in that** the light emitted by the light source is able to pass through the interior of the hollow organ in the area of the measuring gap (14).

15. The device for hemorrhage detection according to any one of claims 1 to 14, **characterized in that** the fixing means (2) constitutes a means for stanching blood of a bleeding source, e.g. is an endoscopic hemostasis clip.

## Revendications

1. Dispositif pour la détection d'une hémorragie, comportant
un dispositif de fixation (2), pouvant être fixé à l'intérieur d'un organe creux,
un dispositif de détection (3) relié au dispositif de fixation (2), permettant de détecter la présence de sang,
une source lumineuse (12), réalisée de manière à émettre de la lumière dans la plage ultraviolette et dans la plage rouge du spectre visible,
au moins un capteur (13) sensible à la lumière, réalisé de manière à mesurer la lumière dans ladite plage ultraviolette et dans ladite plage rouge du spectre visible, et à émettre un signal de capteur respectif en fonction de l'intensité lumineuse mesurée dans les deux plages, et
une unité de traitement de signal, réalisée de manière à calculer un quotient des deux signaux de capteur, ledit quotient étant un paramètre déterminant pour la présence de sang.

2. Dispositif pour la détection d'une hémorragie selon la revendication 1, où la source lumineuse (12) émet successivement la lumière dans la plage ultraviolette et la lumière dans la plage rouge de la lumière visible.

3. Dispositif pour la détection d'une hémorragie selon la revendication 1, où deux capteurs (13) sensibles à la lumière sont prévus, dont le premier ne mesure que la lumière dans la plage ultraviolette, et le deuxième que la lumière dans la plage rouge de la lumière visible.

4. Dispositif pour la détection d'une hémorragie selon la revendication 1, comportant en outre une unité d'émission (4, 5, 6, 7) pour l'émission de données saisies par le dispositif de détection (3), et une unité de réception (9) pour la réception des données émises par l'unité d'émission (7).

5. Dispositif pour la détection d'une hémorragie selon la revendication 1 ou 4, **caractérisé en ce que** l'unité d'émission (4, 5, 6, 7) est encapsulée.

6. Dispositif pour la détection d'une hémorragie selon la revendication 4 ou 5, **caractérisé en ce que** l'unité d'émission (4, 5, 6, 7) effectue une transmission sans fil des données vers l'unité de réception (9).

7. Dispositif pour la détection d'une hémorragie selon l'une des revendications 1 à 6, **caractérisé en ce que** le dispositif de fixation (2) est un clip ou une ancre endoscopique.

8. Dispositif pour la détection d'une hémorragie selon l'une des revendications 1 à 6, **caractérisé en ce que** le dispositif de fixation (2) est une structure de type stent.

9. Dispositif pour la détection d'une hémorragie selon l'une des revendications 1 à 8, **caractérisé en ce que** le dispositif de fixation (2) est en matériau biodégradable.

10. Dispositif pour la détection d'une hémorragie selon l'une des revendications 1 à 9, **caractérisé en ce que** le dispositif de fixation (2) est relié au dispositif de détection (3) par un élément de connexion (18).

11. Dispositif pour la détection d'une hémorragie selon la revendication 10, **caractérisé en ce que** l'élément de connexion (18) est en matériau biodégradable.

12. Dispositif pour la détection d'une hémorragie selon la revendication 1, **caractérisé en ce que** la source lumineuse est une LED.

13. Dispositif pour la détection d'une hémorragie selon l'une des revendications 1 à 12, **caractérisé en ce que** le dispositif de détection (3) comporte une fente de mesure (14).

14. Dispositif pour la détection d'une hémorragie selon la revendication 13, **caractérisé en ce que** la lumière émise par la source lumineuse peut traverser l'intérieur de l'organe creux au niveau de la fente de mesure (14).

15. Dispositif pour la détection d'une hémorragie selon l'une des revendications 1 à 14, **caractérisé en ce que** le dispositif de fixation (2) constitue un moyen d'arrêt d'un saignement, et est par exemple un clip d'hémostase endoscopique.
